# EUROPEAN PATENT SPECIFICATION

(11) **EP 2 637 737 B1**
(45) Date of publication and mention of the grant of the patent: **22.03.2017**
(21) Application number: 11788278.7
(22) Date of filing: 09.11.2011
(51) Int. Cl.: A61N 1/372, A61N 1/37, A61N 1/05

(54) **CONNECTIVITY DETECTION AND TYPE IDENTIFICATION OF AN IMPLANTED LEAD FOR AN IMPLANTABLE MEDICAL DEVICE**
KONNEKTIVITÄTSERKENNUNG UND TYPENIDENTIFZIERUNG EINER IMPLANTIERTEN ELEKTRODE FÜR EINE IMPLANTIERBARE MEDIZINISCHE VORRICHTUNG
DÉTECTION DE CONNECTIVITÉ ET IDENTIFICATION DE TYPE D'UNE DÉRIVATION IMPLANTÉE POUR UN DISPOSITIF MÉDICAL IMPLANTABLE

(30) Priority: 12.11.2010 US 945183
(43) Date of publication of application: 18.09.2013
(73) Proprietor: Medtronic, Inc, Minneapolis, MN 55432 (US)
(72) Inventor: SCHOTZKO, Elizabeth, Ann, Elaine Minneasota 55449 (US); YODER, Matthew, Robert, Crystal Minnesota 55427 (US)
(74) Representative: Hughes, Andrea Michelle
(86) International application number: PCT/US2011/059955
(87) International publication number: WO 2012/094058

(56) References cited:
- WO-A1-2009/097224
- WO-A2-2010/126935
- WO-A2-2011/014464
- US-A- 5 431 692
- US-A1- 2004 073 265
- US-A1- 2010 204 766

## Description

### FIELD

The present invention relates generally to implantable medical devices and, more particularly, to such devices functioning with one or more implanted leads for monitoring and/or delivering therapy.

### BACKGROUND

An implantable intravascular lead assembly is often implanted within a patient's body to provide electrical stimulation to the heart. Such lead assemblies may include one or more leads, each having one or more electrical conductors adapted to be suitably connected to a source of electrical energy, which may be a pacemaker or cardioverter/defibrillator. The conductors of each lead are electrically connected to one or more electrodes situated on the lead, with the electrodes adapted to engage endocardial and/or epicardial tissue of the heart and to enable stimulation and sensing functionalities. To that end, the lead assembly may be intravenously inserted through a body vessel, such as a vein, into one or more cardiac chambers, or alternatively, attached to the epicardial surface of the heart. The conductors are generally sealed from body fluids by a biocompatible and bio-stable insulating material.

In a typical lead assembly, the one or more electrodes of the lead(s) may include a tip electrode that is firmly lodged in, and permanently secured to, either the endothelial lining or the epicardial surface of the heart. These lead assemblies are referred to as endocardial or epicardial leads, respectively. Some examples of conventional endocardial and epicardial leads may be found in U.S. Pat. No. 3,348,548 to Chardack, U.S. Pat. No. 3,754,555 to Schmitt, U.S. Pat. No. 3,814,104 to Irnich et al., U.S. Pat. No. 3,844,292 to Bolduc, U.S. Pat. No. 3,974,834 to Kane, U.S. Pat. No. 5,246,014 to Williams, and U.S. Pat. No. 5,397,343 to Smits. Further, a representative defibrillation lead is described in U.S. Pat. No. 6,178,355 to Williams.

With the increased use of multi-chamber pacemakers and defibrillators, such as those that provide bi-atrial or bi-ventricular pacing capabilities, lead assemblies employing multiple leads are generally required to deliver electrical stimulation to various locations within the heart. In positioning such multiple leads within one or more small vessels of the body, it has become even more important to minimize lead and lead connector sizes. As they have become smaller, the leads, in turn, have become increasingly difficult to mark with the appropriate identification, such as manufacturer identification and/or lead model and serial numbers. Consequently, it can become difficult for a physician to determine the condition of a particular lead during implantation of an implantable medical device (IMD) as well as during post-implantation (when assessing effectiveness of the IMD in sensing physiological signals and delivering therapy to patient).

In many cases, such IMDs are configured to function with leads having complex configurations, employing an assortment of electrodes along the axial lengths of the leads. To that end, such lead configurations would allow the IMD to select, and activate or sense with, various combinations of the electrodes. Accordingly, when used for diagnostic sensing and/or therapy delivery, the electrodes can be activated in any of a variety of configurations, enabling significant flexibility and accuracy with respect to sensing and/or pacing vectors programmed via the IMD.

However, with such complex lead configurations, the task of being able to confirm the connectivity of the lead and the electrodes thereon is made just as important for the physician as being able to identify the lead type. To that end, even if a lead was to be identified so that it could be accordingly used for one or more of sensing or therapy purposes, such use would ultimately be thwarted if the lead's connective functionality is compromised.

US 5,431,692, US 2004/073265, WO 2010/126935 and WO 2009/097224 are concerned with identifying leads and lead types.

Embodiments of the invention address the above-noted limitations of current designs.

### SUMMARY

In general, embodiments of the invention are directed to techniques and corresponding apparatus regarding an implantable medical device (IMD) for detecting lead connectivity as well as identifying lead type. Detecting lead connectivity provides, among other information, confirmation that the lead is connected to the IMD and that such connection has good integrity between the IMD and the electrodes of its leads. With positive affirmation regarding lead connectivity, the IMD can in turn accurately identify the lead type. Such connectivity and lead type information gives a physician enhanced confidence in using and/or reprogramming the IMD with respect to such leads.

The invention provides a method according to claim 1 and a system according to claim 9. Preferred embodiments are defined in the dependent claims.

The details of one or more aspects of the disclosure are set forth in the accompanying drawings and the description below. Other features, objects, and advantages of the disclosure will be apparent from the description and drawings, and from the claims.

### BRIEF DESCRIPTION OF THE DRAWINGS

The following drawings are illustrative of particular embodiments of the present invention and therefore do not limit the scope of the invention. The drawings are not to scale (unless so stated) and are intended for use in conjunction with the explanations in the following detailed description. Embodiments of the present invention will hereinafter be described in conjunction with the appended drawings, wherein like numerals denote like elements.
FIG. 1 is a conceptual diagram illustrating an exemplary system that may be used to provide therapy to and/or monitor a heart of a patient in accordance with certain embodiments of the invention.
FIG. 2 is a conceptual diagram illustrating the exemplary implantable medical device (IMD) and the leads of the system shown in FIG. 1 in greater detail.
FIG. 3 is a conceptual diagram illustrating one example of an implantable multi-polar stimulation lead in accordance with certain embodiments of the invention.
FIGS. 4A-4C are transverse cross-sections of exemplary multi-polar stimulation leads, each having two or more electrodes around the circumference of the lead, in accordance with certain embodiments of the invention.
FIG. 5 is a block diagram illustrating an exemplary configuration of the IMD of FIG. 1 in accordance with certain embodiments of the invention.
FIG. 6 is a flow diagram illustrating an exemplary method for detecting connectivity and type of a lead of the IMD of FIG. 5 in accordance with certain embodiments of the invention.
FIG. 7 is a functional block diagram illustrating an exemplary configuration of programmer of FIG. 1.
FIG. 8 is a block diagram illustrating an exemplary system that includes a server and one or more computing devices that are coupled to the IMD and the programmer of FIG. 1 via a network.

### DETAILED DESCRIPTION OF THE PREFERRED EMBODIMENTS

The following detailed description is exemplary in nature and is not intended to limit the scope, applicability, or configuration of the invention in any way. Rather, the following description provides some practical illustrations for implementing exemplary embodiments of the present invention. Examples of constructions, materials, dimensions, and manufacturing processes are provided for selected elements, and all other elements employ that which is known to those of ordinary skill in the field of the invention. Those skilled in the art will recognize that many of the noted examples have a variety of suitable alternatives.

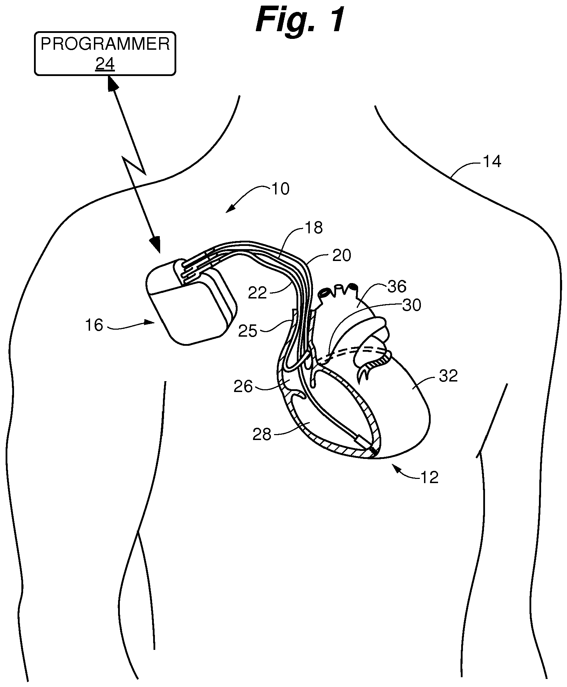

FIG. 1 is a conceptual diagram illustrating an exemplary system 10 that may be used to monitor and/or provide therapy to heart 12 of patient 14 in accordance with certain embodiments of the invention. The patient 14 ordinarily, but not necessarily, will be a human. The system 10 includes an implantable medical device (IMD) 16. The IMD 16 is coupled to one or more leads (e.g., leads 18, 20, and 22) and configured to communicate with a programmer 24 via wireless telemetry. The IMD 16 may be, for example, an implantable pacemaker, cardioverter, and/or defibrillator which provides electrical signals to the heart 12 via electrodes coupled to one or more of the leads 18, 20, and 22.

In accordance with this disclosure, the IMD 16 is configured to communicate with one or more of the leads 18, 20, and 22 to detect lead connectivity as well as to identify lead type. Detecting lead connectivity provides, among other information, confirmation that the lead is connected to the IMD 16 and that such connection has good integrity between the IMD 16 and the electrodes of its leads, as is further detailed herein. With positive affirmation regarding lead connectivity, the IMD 16 can in turn accurately identify the lead type. As described above, given the complex lead configurations commercially available and their differing monitoring and/or therapy functionalities, a varying number of monitoring and/or therapy implementations can be imparted therefrom. For example, two or more electrodes, and the polarity of such electrodes, are used in defining a vector, or path, for delivering pacing pulses to the heart 12. As will be further described herein, with the multiple leads 18, 20, and 22 each configured with one or more electrodes, activation of alternate electrodes located at the same lead area (e.g., on the same electrode band but offset on the band) can vary the pacing vectors. To that end, certain of these pacing vectors may be known to be more effective than others, particularly in light of a given patient's condition and medical history. Confirming lead connectivity and identifying the lead type via the IMD 16, and communicating such information via the programmer 24, avails the physician to confidently use, and reprogram if necessary, the IMD 16 with regard to such pacing vectors.

As shown, the leads 18, 20, 22 extend into the heart 12 of patient 14 to sense electrical activity of the heart 12 and/or deliver electrical stimulation to the heart 12. In the system shown in FIG. 1, right ventricular (RV) lead (referenced as lead 18) extends through one or more veins (not shown), the superior vena cava 25, and right atrium 26, and into right ventricle 28 of the heart 12. Left ventricular (LV) coronary sinus lead (referenced as lead 20) extends through one or more veins, the superior vena cava 25, the right atrium 26, and into the coronary sinus 30 to a region adjacent to the free wall of left ventricle 32 of the heart 12. Right atrial (RA) lead (referenced as lead 22) extends through one or more veins and the superior vena cava 25, and into the right atrium 26 of the heart 12.

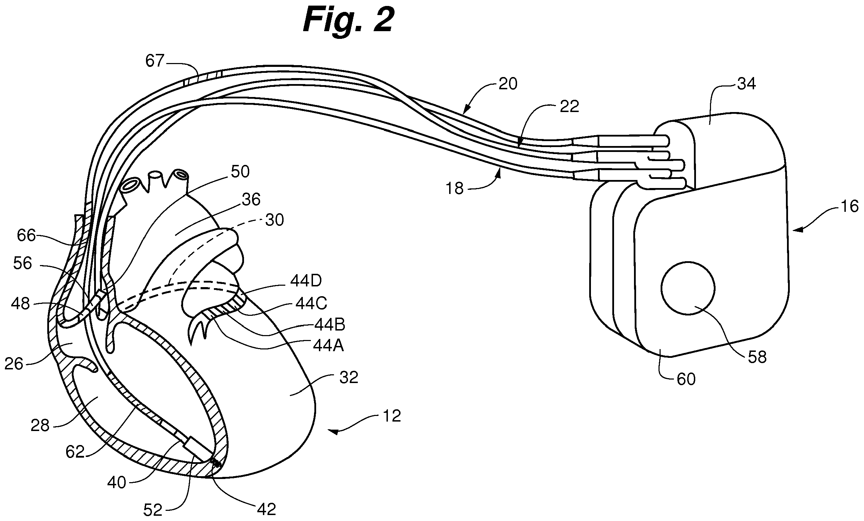

In use, the IMD 16 may sense electrical signals attendant to the depolarization and repolarization of the heart 12 via electrodes (shown in FIGS. 1 and 2, yet only referenced in FIG. 2), at least one of which is coupled to one of the leads 18, 20, 22. In some examples, the IMD 16 provides pacing pulses to the heart 12 based on the electrical signals sensed within the heart 12. The configurations of electrodes used by IMD 16 for sensing and pacing may be unipolar or multi-polar. The IMD 16 may also provide defibrillation therapy and/or cardioversion therapy via electrodes, at least one of which is located on one of the leads 18, 20, 22. The IMD 16 may detect arrhythmia of the heart 12, such as fibrillation of the ventricles 28 and 32 or atrium 26, and deliver defibrillation therapy to the heart 12 in the form of electrical pulses. In some examples, the IMD 16 may be programmed to deliver a progression of therapies, e.g., pulses with increasing energy levels, until a fibrillation of the heart 12 is stopped. In such cases, the IMD 16 can detect fibrillation employing one or more fibrillation detection techniques known in the art.

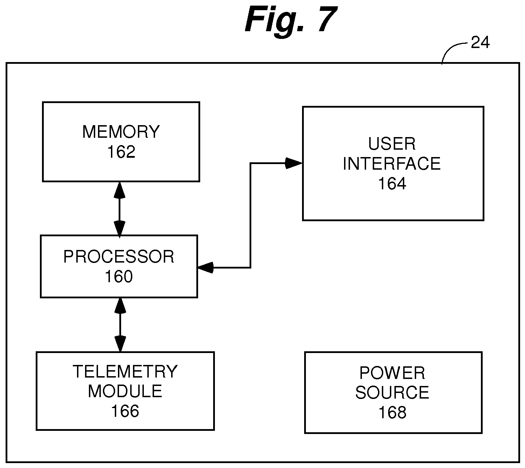

In certain embodiments, as described above, the IMD 16 is configured to communicate wirelessly with the programmer 24 (shown in greater detail in FIG. 7), which may be a handheld computing device or a computer workstation. A physician (which could just as well be a technician, clinician, or other user) may, in turn, interact with the programmer 24 to communicate with the IMD 16. For example, the physician may interact with the programmer 24 to retrieve physiological or diagnostic information from the IMD 16. The physician may also interact with the programmer 24 to reprogram the IMD 16, e.g., select values for operational protocols of the IMD 16.

For example, the physician may use the programmer 24 to retrieve information from the IMD 16 regarding rhythm of the heart 12, trends therein over time, or arrhythmic episodes. As another example, the physician may use the programmer 24 to retrieve information from the IMD 16 regarding other sensed physiological parameters of the patient 14, such as intracardiac or intravascular pressure, activity, posture, respiration, or thoracic impedance. As another example, the physician may use the programmer 24 to retrieve information from the IMD 16 regarding the performance or integrity of the IMD 16 or other components of the system 10, such as the leads 18, 20 and 22, or a power source of the IMD 16. The physician may use the programmer 24 to program a therapy progression, select electrodes used to deliver defibrillation pulses, select waveforms for the defibrillation pulse, and/or select or configure a fibrillation detection algorithm for the IMD 16. The physician may also use the programmer 24 to program aspects of other therapies provided by the IMD 16, such as cardioversion or pacing therapies.

The IMD 16 and programmer 24 may communicate via wireless communication using any techniques known in the art. Examples of communication techniques may include, for example, low frequency or radiofrequency (RF) telemetry, but other techniques are also contemplated. In some examples, the programmer 24 may include a programming head that may be placed proximate to the patient's body near the IMD 16 implant site in order to improve the quality or security of communication between the IMD 16 and the programmer 24.

Using various techniques of this disclosure, a physician will be apprised of the connectivity status and types of one or more of the leads 18, 20, and 22 during implantation of the IMD 16 or during post-implantation (e.g., during routine appointments for assessing the condition of the patient 14 and the to-date functioning of the system 10 in treating such condition). To that end, with such connectivity status and lead type information, the physician during implantation can use the leads with confidence with regard to the sensing and therapy-delivery protocols of the IMD 16 via configurations of electrodes on the leads 18, 20, and 22. Alternately, during post-implantation, these protocols can be adjusted/updated following examination of the patient 14 and download of data from the IMD 16. To that end, in availing the physician of the connectivity status and type of leads during such examinations, not only can the physician have a better appreciation of the validity of the data from the IMD 16, but is in a better position to evaluate the state of the leads 18, 20, and 22 for updating the programmed sensing/therapy protocols of the IMD 16.

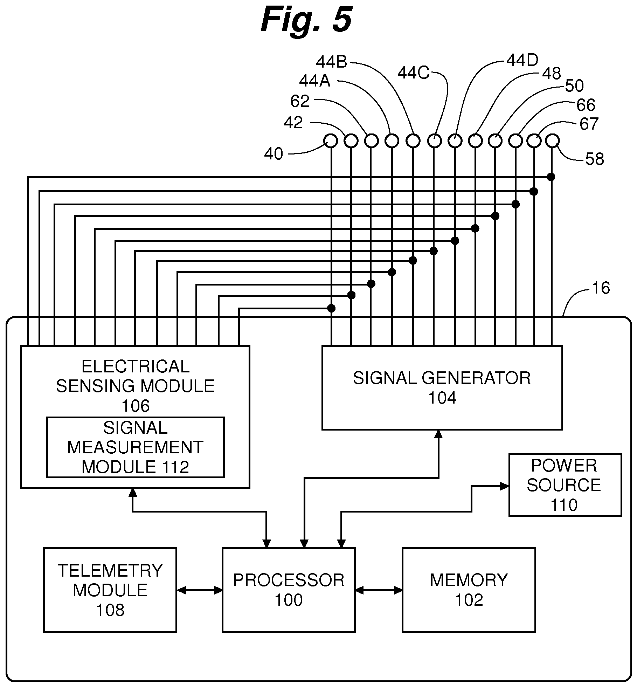

FIG. 2 is a conceptual diagram illustrating the IMD 16 and the leads 18, 20, and 22 of therapy system 10 of FIG. 1 in greater detail. The leads 18, 20, 22 may be electrically coupled to a signal generator and a sensing module of the IMD 16 (as further described herein with respect to FIG. 5) via connector block 34. Each of the leads 18, 20, 22 includes an elongated insulative lead body carrying one or more conductors. In certain embodiments, electrodes 40 and 42 are located adjacent to a distal end of the lead 18, and electrodes 48 and 50 are located adjacent to a distal end of the lead 22. In some example configurations, the lead 20 may be a quadripolar lead and, as such, include four electrodes, namely electrodes 44A-44D, which are located adjacent to a distal end of the lead 20. The electrodes 40, 44A-44D, and 48 may take the form of ring electrodes, and electrodes 42 and 50 may take the form of extendable helix tip electrodes mounted retractably within insulative electrode heads 52 and 56, respectively.

The leads 18 and 22 also include elongated intracardiac electrodes 62 and 66 respectively, which may take the form of a coil (RV coil 62 and RA coil 66). In addition, one of the leads 18, 20, 22, e.g., lead 22 as seen in FIG. 2, may include a superior vena cava (SVC) coil 67 for delivery of electrical stimulation, e.g., transvenous defibrillation. For example, the lead 22 may be inserted through the superior vena cava 25, with the SVC coil 67 being placed, for example, at the right atrial/SVC junction (low SVC) or in the left subclavian vein (high SVC). Each of the electrodes 40, 42, 44A-44D, 48, 50, 62, 66 and 67 may be electrically coupled to a respective one of the conductors within the lead body of its associated lead 18, 20, 22, thereby being individually coupled to the signal generator and sensing module of the IMD 16.

In some examples, as illustrated in FIG. 2, the IMD 16 includes one or more housing electrodes, such as housing electrode 58, which may be formed integrally with an outer surface of hermetically-sealed housing 60 of the IMD 16 or otherwise coupled to the housing 60. In some examples, the housing electrode 58 is defined by an uninsulated portion of an outward facing portion of the housing 60 of IMD 16. Other division between insulated and uninsulated portions of the housing 60 may be employed to define two or more housing electrodes. In some examples, the housing electrode 58 comprises substantially all of the housing 60.

The IMD 16 may sense electrical signals attendant to the depolarization and repolarization of the heart 12 via the electrodes 40, 42, 44A-44D, 48, 50, 58, 62, 66 and 67. The electrical signals are conducted to the IMD 16 via the respective leads 18, 20, 22, or in the case of the housing electrode 58, a conductor coupled to the housing electrode 58. The IMD 16 may sense such electrical signals via any bipolar combination of electrodes 40, 42, 44A-44D, 48, 50, 62, 66 and 67. Furthermore, any of the electrodes 40, 42, 44A-44D, 48, 50, 62, 66 and 67 may be used for unipolar sensing in combination with the housing electrode 58.

In some examples, the IMD 16 delivers pacing pulses via bipolar combinations of the electrodes 40, 42, 44A-44D, 48 and 50 to produce depolarization of cardiac tissue of the heart 12. In some examples, the IMD 16 delivers pacing pulses via any of the electrodes 40, 42, 44A-44D, 48, and 50 in combination with the housing electrode 58 in a unipolar configuration. For example, the electrodes 40 or 42 in combination with the housing electrode 58 may be used to deliver RV pacing to the heart 12. Additionally or alternatively, any of the electrodes 44A-44D may be used in combination with the housing electrode 58 or the RV coil 62 to deliver LV pacing to the heart 12, and the electrodes 48 or 50 in combination with the housing electrode 58 may be used to deliver RA pacing to the heart 12.

Furthermore, the IMD 16 may deliver defibrillation pulses to the heart 12 via any combination of the elongated electrodes 62, 66 and 67, and housing electrode 58. The electrodes 58, 62, and 66 and 67 may also be used to deliver cardioversion pulses to the heart 12. The electrodes 62, 66 and 67 may be fabricated from any suitable electrically conductive material, such as, but not limited to, platinum, platinum alloy or other materials known to be usable in implantable defibrillation electrodes.

The configuration of therapy system 10 illustrated in FIGS. 1 and 2 is merely one example. In other examples, a therapy system may include one or more epicardial leads and/or patch electrodes instead of or in addition to the transvenous leads 18, 20, and 22 illustrated in FIGS. 1 and 2. Further, the IMD 16 need not be implanted within the patient 14. In examples in which the IMD 16 is not implanted in the patient 14, the IMD 16 may deliver defibrillation pulses and other therapies to the heart 12 via percutaneous leads that extend through the skin of the patient 14 to a variety of positions within or outside of the heart 12.

In addition, in other examples, a therapy system may include any suitable number of leads coupled to the IMD 16, and each of the leads may extend to any location within or proximate to the heart 12. For example, other examples of therapy systems may include three transvenous leads located as illustrated in FIGS. 1 and 2, and an additional lead located within or proximate to the left atrium 36.

As described above, two or more electrodes, and the polarity of the electrodes, are used in defining a vector, or path, for delivering pacing pulses to the heart 12. As further described, there are numerous vectors that may be used to deliver pacing pulses to the heart 12. For example, various combinations of the electrodes on a single quadripolar lead (i.e., a lead with four electrodes on the lead, such as the lead 20), as well as combinations of the lead electrodes with an electrode on the housing of the IMD 16, may provide sixteen or more different vectors that may be used to deliver pacing pulses to a chamber of the heart 12 that the lead is within or on. Accordingly, based on the different pacing vectors that can implemented via the varied electrode configurations, the sensing and/or therapy protocols programmed for the patient 14 can be suitably adjusted, based on change in the patient's condition and/or medical history, as well as to-date performance of the IMD 16 in treating the patient's condition.

Confirming lead connectivity and identifying the lead type, and in turn, communicating such information via the programmer 24, avails the physician to use the IMD 16 most effectively during implantation. Further, using such information in light of the sensed physiologic data previously stored by the IMD 16 avails the physician to reprogram the IMD 16 most effectively during post-implantation (e.g., during routine appointments for assessing the patient's condition and to-date functioning of the system 10 in treating such condition). To that end, in order to confirm a most effective pacing vector, and sequence of pacing, is ultimately used, the IMD 16, in certain embodiments, is configured to allow a physician to test a portion of the available criteria and vectors. Such techniques are taught in co-pending U.S. application entitled "Prioritized Programming of Multi-Electrode Pacing Leads," the teachings of which are incorporated herein in relevant part. Upon confirming a most effective pacing vector and/or sequence of pacing for the patient 14, the IMD 16 can be reprogrammed accordingly.

The techniques for confirming lead connectivity and identifying the lead type alluded to above can be described with respect to a multi-electrode lead, such as quadripolar lead 20 of FIG. 2. However, the techniques may also be applied to other multi-polar leads, as shown and described in more detail below.

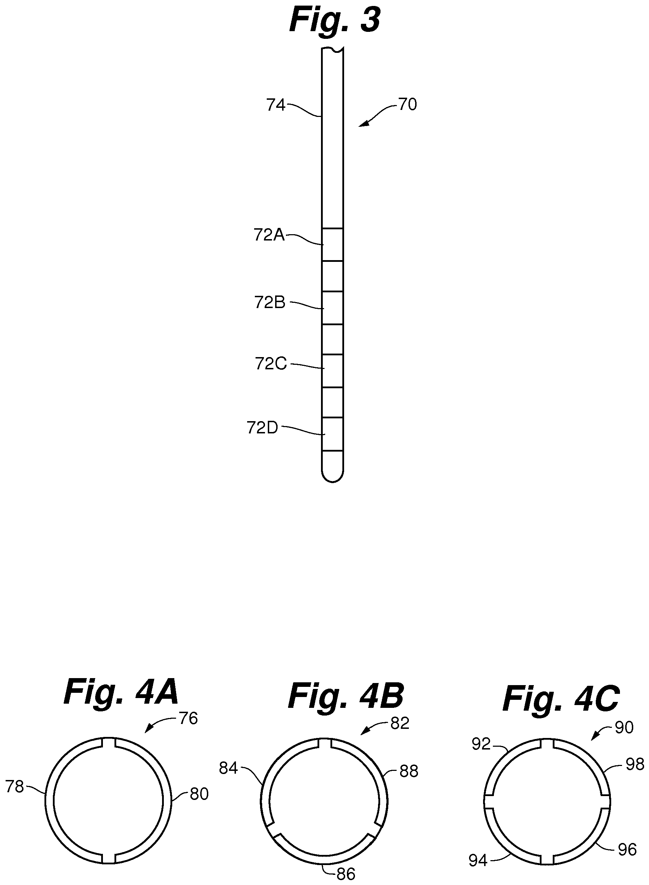

FIG. 3 is a conceptual diagram illustrating one example of an implantable multi-polar stimulation lead in accordance with certain embodiments of the invention. In particular, lead 70 is an example of a multi-polar lead that includes four electrode levels, or bands 72 (exemplarily represented as bands 72A-72D) mounted at various positions along the axial length of lead housing 74. The bands 72A, 72B, 72C, and 72D may be equally spaced along a distal portion of the axial length of the lead housing 74, e.g., as illustrated in FIG. 3. Each of the bands 72 may have two or more electrodes located at different angular positions around the circumference of the lead housing 74, as shown and described below with respect to FIGS. 4A-4C.

FIGS. 4A-4C are transverse cross-sections of exemplary multipolar stimulation leads having two or more electrodes around the circumference of the lead in accordance with certain embodiments of the invention. FIG. 4A shows band 76, which includes two electrodes 78 and 80. In certain embodiments, each of the electrodes 78 and 80 wraps approximately 170 degrees around the circumference of the band 76. In turn, spaces of approximately 10 degrees are located between the electrodes 78 and 80 to prevent inadvertent coupling of electrical current between the electrodes. Either of the electrodes 78 and 80 may be programmed to act as an anode or cathode, and/or the electrodes 78 and 80 may be combined to make a larger electrode.

FIG. 4B shows band 82, which includes three equally sized electrodes 84, 86, and 88. In certain embodiments, each of the electrodes 84, 86 and 88 encompasses approximately 110 degrees of the circumference of the band 82. In turn, similar to the band 76 described above, spaces of approximately 10 degrees separate the electrodes 84, 86 and 88. Any of the electrodes 84, 86 and 88 may be independently programmed as anode or cathode for stimulation, and/or two or more of the electrodes 84, 86, and 88 may be combined together to make a larger electrode.

FIG. 4C shows band 90, which includes four electrodes 92, 94, 96, and 98. In certain embodiments, each of the electrodes 92, 94, 96, and 98 covers approximately 80 degrees of the circumference, with approximately 10 degrees of insulation space between adjacent electrodes. Any of the electrodes 92, 94, 96, and 98 may be independently programmed as an anode or cathode for stimulation, and/or two or more of the electrodes 84, 86, and 88 may be combined together to make a larger electrode.

With further reference to the electrode configurations of exemplary bands of FIGS. 4A-4C, in other embodiments, up to ten or more electrodes may be included within an electrode band. In alternative embodiments, consecutive bands of the lead 70 of FIG. 3 may include a variety of bands (such as any combination of bands 76, 82, and 90 of FIGS. 4A-4C), and the lead 70 may include more bands then are shown in FIG. 3. The above-described sizes of electrodes within a band are merely examples, and various techniques of this disclosure are not limited to the example electrode sizes. For example, one or more of the electrode bands may be of a differing size than one or more of the other electrode bands, and/or the electrode bands may be spaced apart as applicable (e.g., adjacent bands having differing offset distances along the lead's axial length).

Multipolar stimulation leads having two or more electrodes around the circumference of the lead, as in FIGS. 4A-4C, for example, may be useful in not only producing a more effective pacing vector for the patient, but also may be configured for minimizing or eliminating unwarranted stimulation to certain areas, e.g., such as to the phrenic nerve. For example, the IMD 16 can be initially used or reprogrammed by the physician to use one or more electrodes of a band, e.g., the electrodes 92, 94, and 96 of band 90 in FIG. 4C, based on a most effective pacing vector for the patient. However, the selection of the pacing vector can be further based so as to not result in phrenic nerve stimulation. In using multipolar stimulation leads having one or more bands thereon, as exemplified in FIGS. 3 and 4A-4C above, the physician is provided a great degree of flexibility and accuracy to accordingly pinpoint the effect of such pacing vector. However, programming such most effective pacing vector is altogether presumptively based on confirmation of the type and connectivity of the corresponding lead from the IMD 16.

In accordance with certain techniques of this disclosure, a processor of the IMD 16, e.g., processor 100 as further described below in FIG. 5, communicates with one or more of the leads 18, 20, and 22 to determine corresponding connectivity and type of the leads. In this manner, the processor will automatically receive such information relating to the leads and can provide such information to the physician (via the programmer 24) so that most effective pacing vector protocols can be used or reprogrammed with respect to the IMD 16, resulting in corresponding pacing stimuli to be delivered to the heart 12 as needed.

In certain embodiments, one or more of the leads 18, 20, and 22 can include active electronics (not visibly shown) incorporated therewith and capable for controlling a plurality of electrodes on the lead. Such active electronics incorporated with any one of the leads 18, 20, and 22 involves one or more modular circuits incorporated therewith, whether the lead is unipolar or multipolar. In certain embodiments, the modular circuits are incorporated internal to the lead. As described, when used, each of the modular circuits is capable for controlling a plurality of electrodes on the lead. Representative lead configurations employing such active electronics are described in U.S. Pat. No. 7,713,194 to Zdeblick.

Such modular circuits are controlled by sending signals over first and second conduction paths (e.g., the lead conductors) generally connecting back to the IMD 16, which typically provides power and includes control circuitry for the circuits. Each of the modular circuits includes circuitry that is electrically connected to the first and second conduction paths and, as described above, further connected to one or more electrodes of the lead. As such, each of the modular circuits of a lead acts as an interface between the IMD 16 and the electrodes the circuit is connected to. To that end, in one exemplary design, a lead configuration may accommodate eight modular circuits, with each circuit controlling four electrodes on the lead (however, such configurations can be varied as is desired). Such exemplary lead configuration would allow the IMD 16 to select, and activate or sense with, various combinations of the 32 electrodes at any of a variety of sequences. Accordingly, when used for diagnostic sensing and/or therapy delivery, the electrodes of the modular circuits can be activated in an assortment of configurations, enabling significantly increased efficiency over leads not employing such modular circuits.

FIG. 5 is a block diagram illustrating one exemplary configuration of the IMD 16 in accordance with certain embodiments of the invention. In the example illustrated, the IMD 16 includes a processor 100, memory 102, signal generator 104, electrical sensing module 106, telemetry module 108, and a power source 110. Using various techniques of this disclosure, the processor 100 may initiate communication between one or more of the leads 18, 20, and 22 to determine lead connectivity and lead type. In turn, the processor 100 can relay such information from the IMD 16 to the programmer 24 via wireless telemetry for programming purposes by a physician.

The memory 102 may include computer-readable instructions that, when executed by the processor 100, result in the IMD 16 and processor 100 to perform various functions attributed throughout this disclosure, e.g., with respect to communicating with one or more of the leads 18, 20, and 22, as well as the programmer 24. The computer-readable instructions may be encoded within the memory 102. The memory 102 may comprise computer-readable storage media including any volatile, nonvolatile, magnetic, optical, or electrical media, such as a random access memory (RAM), read-only memory (ROM), non-volatile RAM (NVRAM), electrically-erasable programmable ROM (EEPROM), flash memory, or any other digital media.

The power source 110 may be a non-rechargeable primary cell battery or a rechargeable battery and may be coupled to power circuitry. However, the disclosure is not limited to examples in which the power source is a battery. In another example, the power source 110 may comprise a supercapacitor. In some examples, the power source 110 may be rechargeable via induction or ultrasonic energy transmission, and include an appropriate circuit for recovering transcutaneously received energy. For example, the power source 110 may be coupled to a secondary coil and a rectifier circuit for inductive energy transfer. In additional examples, the power source 110 may include a small rechargeable circuit and a power generation circuit to produce the operating power. In further examples, the power source 110 may be coupled to an external power source, for example, in external pacemaker applications.

The processor 100 may include any one or more of a microprocessor, a controller, a digital signal processor (DSP), an application specific integrated circuit (ASIC), a field-programmable gate array (FPGA), or equivalent discrete or integrated logic circuitry. In some examples, the processor 100 may include multiple components, such as any combination of one or more microprocessors, one or more controllers, one or more DSPs, one or more ASICs, or one or more FPGAs, as well as other discrete or integrated logic circuitry. The functions attributed to the processor 100 herein may be embodied as software, firmware, hardware or any combination thereof.

The processor 100 controls the signal generator 104 to deliver stimulation therapy, e.g., cardiac pacing or CRT, to the heart 12 according to one or more therapy protocols programmed by the physician, which may be stored in the memory 102. The signal generator 104 is electrically coupled to the electrodes 40, 42, 44A-44D, 48, 50, 58, 62, 66, and 67, e.g., via conductors of the respective lead 18, 20, 22, or, in the case of the housing electrode 58, via an electrical conductor disposed within the housing 60 of the IMD 16. The signal generator 104 is configured to generate and deliver electrical stimulation therapy to the heart 12 via selected combinations of the electrodes 40, 42, 44A-44D, 48, 50, 58, 62, 66, and 67. In some examples, the signal generator 104 is configured to deliver cardiac pacing pulses. In other examples, the signal generator 104 may deliver pacing or other types of stimulation in the form of other signals, such as sine waves, square waves, or other substantially continuous time signals.

The signal generator 104 may include a switch module (not shown) and the processor 100 may use the switch module to select, e.g., via a data/address bus, which of the available electrodes are used to deliver pacing pulses. The processor 100 may also control which of the electrodes 40, 42, 44A-44D, 48, 50, 58, 62, 66, and 67 is coupled to the signal generator 104 for generating stimulus pulses, e.g., via the switch module. The switch module may include a switch array, switch matrix, multiplexer, or any other type of switching device suitable to selectively couple a signal to selected electrodes. In alternate examples, if the IMD 16 is configured with leads employing active electronics, i.e., modular circuits as described above, the processor 100 may control one or more of the modular circuits which, in turn, enables coupling of the electrodes 40, 42, 44A-44D, 48, 50, 58, 62, 66, and 67 to the signal generator 104 for generating stimulus pulses.

The electrical sensing module 106 monitors signals from at least one of the electrodes 40, 42, 44A-44D, 48, 50, 58, 62, 66, or 67 in order to monitor electrical activity of the heart 12. The electrical sensing module 106 may also include a switch module to select which of the available electrodes are used to sense the cardiac activity. In some examples, the processor 100 selects the electrodes that function as sense electrodes, or the sensing vector, via the switch module within electrical sensing module 106. In alternate examples, if the IMD 16 is configured with leads employing active electronics, i.e., modular circuits as described above, the processor 100 may control one or more of the modular circuits which, in turn, enable the selected electrodes to function as sense electrodes, or the sensing vector, for the sensing module 106.

The electrical sensing module 106 includes multiple detection channels, each of which may be selectively coupled to respective combinations of the electrodes 40, 42, 44A-44D, 48, 50, 58, 62, 66, or 67 to detect electrical activity of a particular chamber of the heart 12. Each detection channel may comprise an amplifier that outputs an indication to the processor 100 in response to detection of an event, such as a depolarization, in the respective chamber of the heart 12. In this manner, the processor 100 may detect the occurrence of R-waves and P-waves in the various chambers of the heart 12. Similarly, the processor 100 may also detect other sensed physiological parameters of the patient 14, such as intracardiac or intravascular pressure, activity, posture, respiration, or thoracic impedance in known fashions.

The memory 102 is used for storing data used by the processor 100 to control the delivery of pacing pulses by the signal generator 104. Such data may include intervals and counters used by processor 100 to control the delivery pacing pulses to one or both of the left and right ventricles for CRT. The intervals and/or counters are, in some examples, used by processor 100 to control the timing of delivery of pacing pulses relative to an intrinsic or paced event, e.g., in another chamber.

In certain embodiments, the IMD 16 is further equipped with means to make measurements of signals detected by the sensing module 106. As further described below, these measurements can in turn be processed to impart one or more parameters relating to the leads 18, 20, and 22 of the IMD 16 from the detected signals. Such measurement means may be implemented as a further module of the IMD 16, i.e., signal measurement module 112. In certain embodiments, as shown, such signal measurement module 112 may be configured as part of the electrical sensing module 106. However, the invention should not be limited to such. For example, the signal measurement module 112 may be configured apart from the sensing module 106 and/or may be implemented as part of the processor 100.

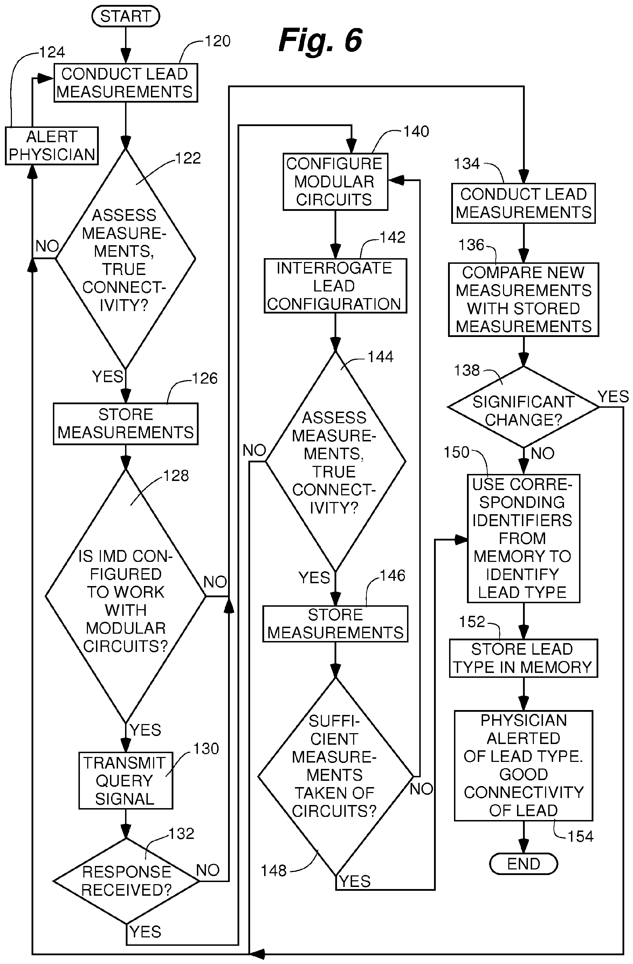

As further described below with respect to FIG. 6, the signal measurement module 112 is used in evaluating connectivity of the leads 18, 20, and 22. For example, impedance is one criterion by which the lead connectivity may be evaluated using the module 112; however, other parameters from signals detected by the sensing module 106, such as signal noise, may be measured to, in turn, impart lead connectivity. In the case of lead impedance, the signal measurement module 112 is configured for measuring signals detected from two or more of the electrodes used for a pacing vector. In some examples, impedance may be measured for any of a variety of electrical paths that include two or more electrodes of the leads 18, 20, 22, or one of the those electrodes and the IMD housing electrode 58, e.g., at least one of electrodes 44A-44D in combination with one of electrodes 40, 42, 48, 50, 58, 62, 66, and 67. In the illustrated example of FIG. 5, the sensing module 106 incorporates the signal measurement module 112, which may measure electrical parameter values during delivery of an electrical signal between at least two of the electrodes. The processor 100 may control signal generator 104 to deliver the electrical signal between the electrodes. In turn, the processor 100 may determine impedance values based on parameter values measured by the signal measurement module 112, and store measured impedance values in the memory 102.

In the case of measuring lead impedance, some examples may involve the processor 100 performing an impedance measurement by controlling delivery, from signal generator 104, of a voltage pulse between first and second electrodes. As described above, when using leads implemented with modular circuits, the processor 100 controls such circuits in enabling delivery of such voltage pulse. The signal measurement module 112 may measure a resulting current, and the processor 100 may derive an impedance value there from, e.g., based upon the voltage amplitude of the pulse and the measured amplitude of the resulting current.

In other examples, the processor 100 may perform an impedance measurement by controlling delivery, from signal generator 104, of a current pulse between first and second electrodes. Similar to that described above, when using leads implemented with modular circuits, the processor 100 controls such circuits in enabling delivery of such current pulse. The measurement module 112 may measure a resulting voltage, and the processor 100 may derive an impedance value there from, e.g., based upon the current amplitude of the pulse and the measured amplitude of the resulting voltage. The measurement module 112 may include circuitry for measuring amplitudes of resulting currents or voltages, such as sample and hold circuitry.

To that end, in certain cases of measuring impedance, the IMD 16 may collect impedance values that include both a resistive and a reactive (i.e., phase) component. In such cases, the IMD 16 may measure impedance during delivery of a sinusoidal or other time varying signal by the signal generator 104, for example. Thus, as used in this disclosure, the term "impedance" is used in a broad sense to indicate any collected, measured, and/or calculated value that may include one or both of resistive and reactive components.

FIG. 6 is a flow diagram illustrating an exemplary method for detecting connectivity and type of a lead of the IMD 16 in accordance with certain embodiments of the invention. As should be appreciated from the above, the IMD 16 is configured to function with leads 18, 20, and 22, either involving standard leads (those without modular circuits therein) or multi-polar leads incorporated with modular circuits as described above. To that end, the standard leads can be uni-polar or multi-polar, yet not be modular circuit-enabled. In certain embodiments, as described below, the IMD 16 is configured to detect the connectivity/integrity of these leads, i.e., to confirm the IMD 16 is properly connected to the lead and its electrodes, using a similar series of steps. In turn, the IMD 16 interrogates the lead in determining its type so that the correct functionality can be enabled for the lead.

Using the IMD 16 and its exemplary lead system (involving leads 18, 20, and 22), the method (performed by an algorithm of the processor 100 of the IMD 16) begins when the leads 18, 20, and 22 are already implanted and operatively coupled to the IMD 16 (via its connector block 34). As described above, the first stage of the method involves steps in which the connectivity of one or more of the leads 18, 20, and 22 is confirmed. As described above, in certain embodiments, this involves taking measurements in relation to one of the leads 18, 20, and 22. As such, an initial step 120 of such first stage involves conducting lead measurements, and in this described example, the measurements are made with regard to the lead 20.

In certain embodiments, conducting lead measurements in step 120 involves measuring one or more parameters (e.g., voltage or current) with respect to a plurality of pairings of the lead electrodes 44A-44D and/or with respect to single of the lead electrodes 44A-44D and the housing electrode 58. In turn, the processor 100 uses such parameters to derive the lead measurements. However, it should be appreciated that in some examples, such parameters may provide sufficient information regarding connectivity of the lead 20 so as to not require further derivation of the measured parameters. As described above, in certain embodiments, when conducting lead measurements in step 120, impedance values of the lead 20 are ultimately determined; however, the invention need not be limited to impedance as being the only variable that can be used in assessing connectivity/integrity of the lead 20.

In certain embodiments, in the course of conducting lead measurements in step 120, the IMD processor 100 initially retrieves instructions from the memory 102 for controlling the signal generator 104. With regard to making impedance measurements, in certain embodiments, the processor 100 controls the signal generator 104 to perform a pacing function. Such pacing function involves transmitting pulses at a threshold not significant enough to actually stimulate the tissue, but instead at a lower threshold from which corresponding parameters can still be detected.

As described above, performing such pacing function involves applying one of a voltage or current pulse along a first conductor of the lead 20 via the signal generator 104 (as controlled by the processor 100). Applying such pacing pulse further serves to activate any modular circuits on the lead 20, if any, so as to further transmit the pulse in relation to the lead electrodes. Transmitting the pulse in relation to different pairings of the electrodes 44A-44D of the lead 20, or in relation to single of the electrodes 44A-44D and the housing electrode 58, results in corresponding parameters that are detected by the sensing module 106. These parameters are in turn measured (e.g., via the signal measurement module 112), from which the lead measurements (e.g., impedance values) are derived (e.g., via the processor 100). Such process is performed in relation to each of the lead electrodes 44A-44D in order to detect the connectivity of the lead 20, as its axial length is segmented between the electrodes 44A-44D thereon.

As described above, in certain embodiments, the signal measurement module 112 is used for measuring corresponding parameters detected (via the sensing module 106) as a result of the pulses passed through the lead electrodes 44A-44D. The frequency of such measurements can be as desired, e.g., beat-to-beat, an alternate frequency, or a user-defined frequency. Further, such measurements can be based off a varied magnitude, e.g., such as a sub-threshold measurement, a pacing pulse measurement, or a supra-threshold measurement. Additionally, the impedance measurements may involve multiple alternative pacing vectors. For example, with regard to pacing vectors to the RV coil 62 and/or housing electrode 58, the impedance measurement may involve a measurement from LV tip 44A to RV coil 62/housing electrode 58, from LV ring 44B or 44C to RV coil 62/housing electrode 58, from LV tip 44A to LV ring 44B or 44C, or other combinations of such electrodes.

Once the lead measurements are conducted (e.g., derived by the processor 100) in step 120, the processor 100 assesses such measurements to determine whether they fall within valid ranges in step 122. In certain embodiments, such valid ranges of the values are held within the IMD memory 102 and retrieved by the processor 100 during the assessment. Regarding impedance measurements made in relation to the electrodes 44A-44D, one valid range is retrieved from the memory 102 for each of the measurements. What can complicate matters is that impedance measurements from pacing vectors for standard leads may be distinct from those same pacing vectors for leads incorporated with modular circuits. Thus, a valid measurement for one lead type may prove to be an invalid measurement for the other. Therefore, in certain embodiments, the valid impedance measurements involve measurements corresponding to standard leads, and measurements taken with respect to the lead 20 involve uni-polar electrode measurements (e.g., in which multi-electrode configurations for any band electrode are detected via the sensing module 106 as a single electrode, with its multiple electrodes tied together in relation to the IMD can 60).

If any of the measured impedance values are found to fall outside the valid range of values, the processor 100 finds connectivity of the lead 20 is not true, and moves to step 124. In step 124, the physician is alerted of the connectivity issue (e.g., by the programmer 24 via wireless communication from the IMD 16). In turn, the physician can attempt to troubleshoot the problem (e.g., disconnecting and reconnecting the lead 20 to the IMD connector block 34), and the process loops back to step 120 to again run through the algorithm's first stage. Conversely, if the measured impedance values are all found to be within the valid range of values, the processor 100 confirms connectivity of the lead 20 is true, and then stores such values within the IMD memory 102 in step 126. In turn, the flowchart moves on to the second stage of the process, involving steps in which lead type is determined.

Step 128 involves determining whether the lead 20 is of a standard design or configured with modular circuits, and to that end, whether the IMD 16 is configured for working with both lead types. If the IMD 16 is configured to work with both lead types, and more particularly, with leads configured with modular circuits, the IMD processor 100 is able to retrieve instructions from the memory 102 for controlling the signal generator 104 to transmit a corresponding query signal for such circuits. In turn, such query signal is transmitted in step 130 along a first conductor of the lead 20 to prompt in-kind responses from the modular circuits, if any are incorporated with the lead 20.

For example, this in-kind response involves each of the modular circuits (in response to the query signal being received) in turn transmitting a signal over a second conductor of the lead 20 to the processor 100 (via the electrical sensing module 106). In certain embodiments, such in-kind response from each of the modular circuits provides the processor 100 information regarding the modular circuit and the electrodes it controls. In certain cases, it is possible that the query signal was not received by one or more of the modular circuits, resulting in fewer in-kind responses being transmitted than the actual number of circuits on the lead 20. Accordingly, in certain embodiments, step 130 can involve sending one or more additional query signals along the lead first conductor. If no in-kind signals are received by the processor 100 after transmitting the one or more additional query signals, the processor 100 will deem the lead 20 to have no modular circuits in step 132, and the process moves to step 134. Further, if the IMD 16 is only configured to work with standard leads, such that no instructions regarding query signal are accessible via the memory 102 in step 128, the process also moves on to step 134.

In step 134, based on the processor 100 determining that the IMD 16 is only configured to work with standard leads or the lead 20 is without modular circuits, measurements with respect to the lead 20 are again conducted (similar to step 120), yet this time, to aid in identifying the lead type. With the lead 20 being without modular circuits, the lead's differing impedances in relation to its electrodes 44A-44D have already been gathered and stored in the IMD memory 102 in steps 120 and 126, respectively. Accordingly, these same impedance measurements are retrieved from the memory 102 by the processor 100 in step 136, and the newly measured parameters are in turn compared with the previously-stored values to confirm there has been no significant change. If there is significant change found in step 138, the process loops to step 124. Conversely, if there is no significant change in the measurements, the electrode configurations of the lead 20, and its type, are identified via the processor 100 using corresponding identifiers stored in the memory 102 in step 150. In certain embodiments, the identifiers can be related to the measured impedances by the processor 100 for identifying the lead type. Once identified, the lead type is stored in the memory 102 in step 152, and the process moves to step 154, as further described below.

Alternately, if the IMD 16 is found to be configured to work with both lead types (prompting a query signal to be transmitted in step 130), and the lead 20 is found to be configured with one or more modular circuits (such that an in-kind response is in turn transmitted by each of the circuits and received by the processor 100 in step 132), the process moves on to step 140. The IMD processor 100, in step 140, retrieves instructions from the memory 102 for transmitting a signal over the lead first conductor (via the signal generator 104) to configure the modular circuits. Such configuration process involves programming active configuration of one or more of the modular circuits, whereby such configuration enables such circuits to in turn be interrogated in step 142. In certain embodiments, such interrogation process can involve similar steps to those of step 120 (in which measurements in relation to the lead 20 are conducted), yet involves lead measurements being made in relation to the differing electrode configurations of the modular circuits. In the interrogation process, the active modular circuits may additionally respond with modular circuit lead type. As should be appreciated, this lead type may be used by the processor 100 to confirm the measurements taken with regard to the active modular circuits, or conversely, may be used by the processor 100 in place of the lead measurements.

For interrogating the lead 20 such that lead measurements are conducted, the signal generator 104, in step 142, applies one of a voltage or current pulse along the first conductor of the lead 20 (as controlled by the processor 110) to electrode pairings of the one or more active modular circuits. In turn, one or more parameters (e.g., voltage or current) with respect to the electrode pairings of the lead electrodes and/or with respect to single of the lead electrodes and the housing electrode 58 are detected by the IMD sensing module 106 and measured by the signal measurement module 112. In turn, the processor 100 uses such parameters to derive the lead measurements. As described above, in certain embodiments, when conducting lead measurements in step 120, impedance values of the lead 20 are ultimately determined, and in certain embodiments, impedance measurements are similarly gathered in step 142. However, the invention need not be limited to impedance being the only variable for assessing connectivity/integrity of the electrodes of the active modular circuits.

Once the lead measurements are conducted (e.g., derived by the processor 100) with respect to the active modular circuits in step 142, the processor 100 assesses such measurements to determine whether they fall within valid ranges in step 144. In certain embodiments, such valid ranges of the values are held within the IMD memory 102 and retrieved by the processor 100 during the assessment. Regarding impedance measurements made in relation to the active modular circuits and their corresponding electrode configurations, one valid range is retrieved from the memory 102 for each of the measurements.

If any of the measured impedance values are found to fall outside the valid range of values, the processor 100 finds connectivity the lead 20 employing the modular circuits is not true. Accordingly, the process loops back to step 124. Conversely, if the measured impedance values are all found to be within the valid range of values, the processor 100 confirms connectivity of the lead 20 is true, and then stores such values within the IMD memory 102 in step 146. Following step 146, further configuration of the modular circuits of the lead 20 may need to occur to fully confirm the connectivity of the lead 20. Accordingly, the flowchart moves to step 148, in which the processor 100 determines whether further interrogation of the modular circuits is necessary for identifying type of the lead 20. If further interrogation is necessary, the process loops back to step 140. However, if no further interrogation is necessary, the process moves to step 150, in which the electrode configurations of the lead 20, and its type, are identified via the processor 100 using corresponding identifiers stored in the IMD memory 102. In certain embodiments, the identifiers can be related to the measured impedances by the processor 100 for identifying the lead type. Subsequently, in step 152, the type of the lead 20 is stored in memory 102, and the process moves to step 154.

In step 154, the physician is alerted of the lead type for lead 20 and its good connectivity (e.g., by the programmer 24 via wireless communication from the IMD 16). In turn, the physician can use or reprogram the IMD 16 with confidence, with the lead type being identified and the lead's connectivity being confirmed.

FIG. 7 is functional block diagram illustrating an example configuration of the programmer 24 in certain embodiments of the invention. As shown, the programmer 24 may include a processor 160, memory 162, user interface 164, telemetry module 166, and power source 168. The programmer 24 may be a dedicated hardware device with dedicated software for programming of the IMD 16. Alternatively, the programmer 24 may be an off-the-shelf computing device running an application that enables the programmer 24 to program the IMD 16.

A physician may use the programmer 24 to select therapy programs (e.g., sets of stimulation parameters), generate new therapy programs, modify therapy programs through individual or global adjustments or transmit the new programs to a medical device, such as the IMD 16 (FIG. 1). The clinician may interact with programmer 24 via user interface 164, which may include display to present graphical user interface to a physician, and a keypad or another mechanism for receiving input from a physician. The physician may define or select vectors to be tested and/or input vector impedance values via the user interface 164.

The user interface 164 may comprise a display screen as well as speakers for outputting an audio signal to the physician. In addition, the programmer 24 may be configured to print measurements, vectors, and the like, or include an interface for connecting the programmer 24 to an output device for printing measurements, vectors, and the like.

The processor 160 can take the form one or more microprocessors, DSPs, ASICs, FPGAs, programmable logic circuitry, or the like, and the functions attributed to the processor 160 herein may be embodied as hardware, firmware, software or any combination thereof. The memory 162 may store instructions that cause the processor 160 to provide the functionality ascribed to the programmer 24 herein, and information used by the processor 160 to provide the functionality ascribed to the programmer 24 herein. The memory 162 may include any fixed or removable magnetic, optical, or electrical media, such as RAM, ROM, CD-ROM, hard or floppy magnetic disks, EEPROM, or the like. The memory 162 may also include a removable memory portion that may be used to provide memory updates or increases in memory capacities. A removable memory may also allow patient data to be easily transferred to another computing device, or to be removed before programmer 24 is used to program therapy for another patient.

The programmer 24 may communicate wirelessly with the IMD 16, such as using RF communication or proximal inductive interaction. This wireless communication is possible through the use of the telemetry module 166, which may be coupled to an internal antenna or an external antenna. An external antenna that is coupled to the programmer 24 may correspond to the programming head that may be placed over the heart 12, as described above with reference to FIG. 1. The telemetry module 166 may be similar to the telemetry module 108 of IMD 16 (FIG. 5).

The telemetry module 166 may also be configured to communicate with another computing device via wireless communication techniques, or direct communication through a wired connection. Examples of local wireless communication techniques that may be employed to facilitate communication between programmer 24 and another computing device include RF communication according to the 802.11 or Bluetooth specification sets, infrared communication, e.g., according to the IrDA standard, or other standard or proprietary telemetry protocols. In this manner, other external devices may be capable of communicating with the programmer 24 without needing to establish a secure wireless connection. An additional computing device in communication with the programmer 24 may be a networked device such as a server capable of processing information retrieved from the IMD 16.

In some examples, the processor 160 of the programmer 24 and/or one or more processors of one or more networked computers may perform all or a portion of the techniques described herein with respect to the processor 160 and the IMD 16. For example, the processor 160 or another processor may receive voltages or currents measured by the IMD 16 to calculate impedance measurements, or may receive impedance measurements from the IMD 16. The power source 168 delivers operating power to the components of the programmer 24.

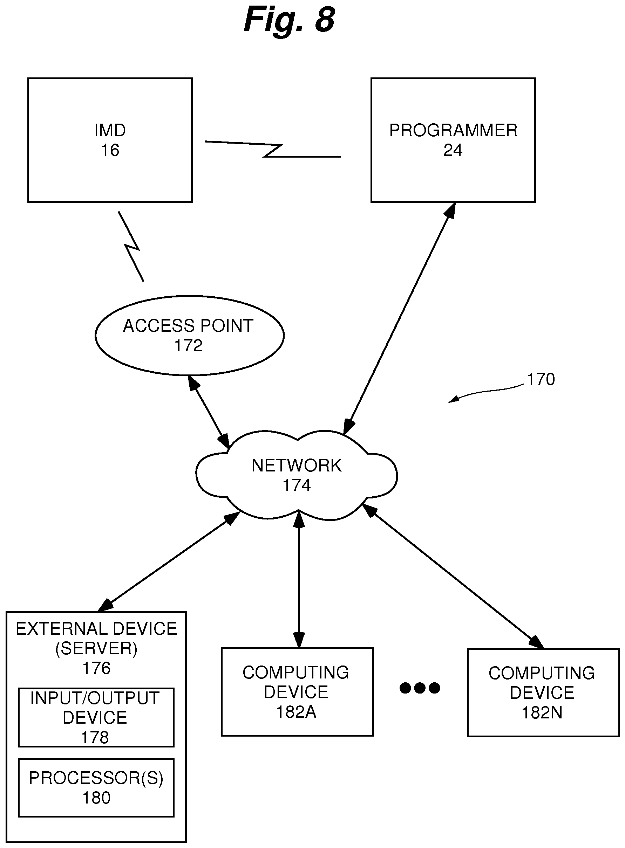

FIG. 8 is a block diagram illustrating an example system 170 that includes an external device, such as a server 176 having an input/output device 178 and processor(s) 180, and one or more computing devices 182A-182N, that are coupled to the IMD 16 and the programmer 24 shown in FIG. 1 via a network 174. In this example, the IMD 16 may use its telemetry module 108 to communicate with the programmer 24 via a first wireless connection, and to communication with an access point 172 via a second wireless connection. In the example of FIG. 8, the access point 172, the programmer 24, the server 176, and the computing devices 182A-182N are interconnected, and able to communicate with each other, through the network 174. In some cases, one or more of the access point 172, the programmer 24, the server 176, and the computing devices 182A-182N may be coupled to the network 174 through one or more wireless connections. The IMD 16, the programmer 24, the server 176, and the computing devices 182A-182N may each comprise one or more processors, such as one or more microprocessors, DSPs, ASICs, FPGAs, programmable logic circuitry, or the like, that may perform various functions and operations, such as those described herein.

The access point 172 may comprise a device that connects to the network 174 via any of a variety of connections, such as telephone dial-up, digital subscriber line (DSL), or cable modem connections. In other examples, the access point 172 may be coupled to the network 174 through different forms of connections, including wired or wireless connections. In some examples, the access point 172 may be co-located with the patient 14 and may comprise one or more programming units and/or computing devices (e.g., one or more monitoring units) that may perform various functions and operations described herein. For example, the access point 172 may include a home-monitoring unit that is colocated with the patient 14 and that may monitor the activity of the IMD 16.

In some cases, the server 176 may be configured to provide a secure storage site for data that has been collected from the IMD 16 and/or the programmer 24. The network 174 may comprise a local area network, wide area network, or global network, such as the Internet. In some cases, the programmer 24 or the server 176 may assemble data in web pages or other documents for viewing by trained professionals, such as clinicians, via viewing terminals associated with computing devices 182A-182N. The illustrated system of FIG. 8 may be implemented, in some aspects, with general network technology and functionality similar to that provided by the Medtronic CareLink® Network developed by Medtronic, Inc., of Minneapolis, MN.

Thus, embodiments of the invention are disclosed. Although the present invention has been described in considerable detail with reference to certain disclosed embodiments, the disclosed embodiments are presented for purposes of illustration and not limitation and other embodiments of the invention are possible. One skilled in the art will appreciate that various changes, adaptations, and modifications may be made without departing from the scope of the invention as defined by the appended claims.

## Claims

1. A method of providing information regarding an implanted lead for an implantable medical device, the method comprising:
conducting, by a processor of an implantable medical device (IMD), one or more measurements in relation to an implanted lead coupled to the IMD;
determining, by the processor, characteristics of the implanted lead based on the one or more measurements; and identifying, by the processor, type of implanted lead based on the lead characteristics, and wherein
the implanted lead comprises one of a lead having one or more modular circuits incorporated therewith and a lead without said modular circuits, wherein each of the modular circuits defines an interface between conductors and corresponding electrodes of the implanted lead, and **characterized in that** the IMD is configured to function with the modular circuits, and **in that** the method further comprises:
transmitting one or more query signals along the lead conductors to prompt in-kind responses transmitted from each of the modular circuits to the processor, wherein the responses comprise electrode configuration information pertaining to the modular circuits.

2. The method of claim 1 wherein conducting the one or more measurements further comprises:
applying pacing pulses corresponding to pacing vectors for electrodes of the implanted lead, wherein each of the pulses is at a threshold not significant enough to stimulate body tissue yet from which a corresponding parameter can be detected; and
deriving the one or more measurements in relation to the corresponding parameters.

3. The method of claim 2 further comprising:
selecting, by the processor, the pacing vectors in relation to a heart.

4. The method of claim 1 wherein conducting the one or more measurements comprises deriving one or more impedance values of the implanted lead.

5. The method of claim 1 wherein conducting the one or more measurements comprises deriving values of an electrical variable of the implanted lead, and further comprising:
assessing, by the processor, whether the derived values of the electrical variable fall within valid ranges of the electrical variable, the valid ranges of the electrical variable stored within memory of the IMD.

6. The method of claim 5 wherein the valid ranges of the electrical variable correspond to the lead characteristics for identifying the lead, wherein the lead characteristics comprise connectivity of the lead conductors between the IMD and electrodes of the lead, and the method further comprising:
associating, by the processor, the derived values of the electrical variable with corresponding lead type identifiers stored within the IMD memory.

7. The method of claim 6 wherein the electrical variable comprises impedance of the implanted lead, and wherein each of the lead type identifiers comprises an electrode configuration of the implanted lead based on a corresponding impedance value of a segment of the implanted lead.

8. The method of claim 1 wherein conducting the one or more measurements comprises deriving an electrical variable of the implanted lead for differing active arrangements of the modular circuits in relation to electrodes thereof.

9. A system that facilitates information regarding an implanted lead for an implantable medical device, the system comprising:
an implantable medical device (IMD) (16);
one or more leads (18, 20, 22) for one or more of sensing activity and delivering electrical stimulation of one or more of tissue and an organ of the patient, the one or more leads implanted in the patient and coupled to the implantable medical device; and
a processor (100) of the IMD configured to:
conduct one or more measurements in relation to one of the implanted leads;
determine characteristics of the one implanted lead based on the one or more measurements; and
identify type of the one implanted lead based on the lead characteristics; wherein the one or more implanted leads comprise one of leads having one or more modular circuits incorporated therewith and leads without said modular circuits, wherein each of the modular circuits defines an interface between conductors and corresponding electrodes of the lead; and **characterized in that** the IMD is configured to function with the modular circuits and **in that** the processor is configured for transmitting one or more query signals along the lead conductors to prompt in-kind responses transmitted from each of the modular circuits to the processor, and **in that** the modular circuits are configured to transmit responses comprising electrode configuration information pertaining to the modular circuits.

10. The system of claim 9 wherein the processor is configured to derive one or more impedance values of the one implanted lead when conducting the one or more measurements.

11. The system of claim 9 wherein the processor is configured to derive values of an electrical variable when conducting the one or more measurements, whereby the processor is further configured to:
assess whether the derived values of the electrical variable fall within valid ranges of the electrical variable, the valid ranges of the electrical variable stored within memory of the IMD; and
associate the derived values of the electrical variable with corresponding lead type identifiers stored within the IMD memory.

12. The system of claim 11 wherein the electrical variable comprises impedance of the implanted lead, and wherein each of the lead type identifiers comprises an electrode configuration of the implanted lead based on a corresponding impedance value of a segment of the implanted lead.

13. The system of claim 9 further comprising a computing device (182) in communication with the IMD for communicating the type of the implanted lead to a user.

## Patentansprüche

1. Verfahren zum Bereitstellen von Information bezüglich einer implantierten Leitung für eine implantierbare medizinische Vorrichtung, wobei das Verfahren umfasst:
Durchführen einer oder mehrerer Messungen durch einen Prozessor einer implantierbaren medizinischen Vorrichtung (IMD) in Bezug auf eine implantierte Leitung, die an die IMD gekoppelt ist;
Bestimmen von Eigenschaften der implantierten Leitung durch den Prozessor, basierend auf der einen oder den mehreren Messungen; und
Identifizieren des Typs der implantierten Leitung durch den Prozessor, basierend auf den Leitungseigenschaften, und wobei
die implantierte Leitung eine Leitung mit einem oder mehreren damit integrierten modularen Schaltkreisen umfasst oder eine Leitung ohne die modularen Schaltkreise umfasst, wobei jeder der modularen Schaltkreise eine Schnittstelle zwischen Stromleitern und entsprechenden Elektroden der implantierten Leitung definiert, und **dadurch gekennzeichnet, dass**
die IMD konfiguriert ist, um mit den modularen Schaltkreisen zusammenzuwirken bzw. zu funktionieren, und dadurch, dass das Verfahren weiter umfasst:
Übertragen eines oder mehrerer Abfragesignale entlang der Leitungsstromleiter, um entsprechende Reaktionen bzw. In-Kind-Reaktionen zu veranlassen, die von jedem der modularen Stromkreise an den Prozessor übertragen werden, wobei die Reaktionen Elektrodenkonfigurationsinformation umfassen, die die modularen Stromkreise betreffen.

2. Verfahren nach Anspruch 1, wobei das Durchführen der einen oder der mehreren Messungen weiterhin umfasst:
Anwenden von Schrittmacherpulsen, die Schrittmachervektoren für Elektroden der implantierten Leitung entsprechen, wobei jeder der Pulse bei einem Schwellwert ist, der nicht signifikant genug ist, um Körpergewebe zu stimulieren, wovon jedoch ein entsprechender Parameter detektiert werden kann; und
Ableiten der einen oder mehreren Messungen in Bezug zu den entsprechenden Parametern.

3. Verfahren nach Anspruch 2, weiter umfassend:
Auswählen der Schrittmachervektoren durch den Prozessor in Bezug auf ein Herz.

4. Verfahren nach Anspruch 1, wobei das Durchführen der einen oder mehreren Messungen das Ableiten eines oder mehrerer Impedanzwerte der implantierten Leitung umfasst.

5. Verfahren nach Anspruch 1, wobei das Durchführen der einen oder mehreren Messungen das Ableiten von Werten einer elektrischen Variable der implantierten Leitung umfasst, und weiter umfasst:
Bewerten durch den Prozessor, ob die abgeleiteten Werte der elektrischen Variable innerhalb von gültigen Bereichen der elektrischen Variable fallen, wobei die gültigen Werte der elektrischen Variable innerhalb eines Speichers der IMD gespeichert sind.

6. Verfahren nach Anspruch 5, wobei die gültigen Bereiche der elektrischen Variable mit den Leitungseigenschaften zum Identifizieren der Leitung korrespondieren, wobei die Leitungseigenschaften die Anschlussfähigkeit der Leitungsstromleiter zwischen der IMD und Elektroden der Leitung umfassen, wobei das Verfahren weiter umfasst:
Assoziieren der abgeleiteten Werte der elektrischen Variable durch den Prozessor mit entsprechenden Leitungstypidentifikatoren, die innerhalb des IMD-Speichers gepeichert sind.

7. Verfahren nach Anspruch 6, wobei die elektrische Variable eine Impedanz der implantierten Leitung aufweist, und wobei jeder der Leitungstypidentifikatoren eine Elektrodenkonfiguration der implantierten Leitung aufweist, die auf einem entsprechenden Impedanzwert eines Segments der implantierten Leitung basiert.

8. Verfahren nach Anspruch 1, wobei das Durchführen der einen oder mehreren Messungen das Ableiten einer elektrischen Variable der implantierten Leitung zum Unterscheiden aktiver Anordnungen der modularen Schaltkreise in Bezug zu den Elektroden davon umfasst.

9. System, das eine Information in Bezug zu einer implantierten Leitung für eine implantierbare medizinische Vorrichtung vereinfacht, wobei das System aufweist:
eine implantierbare medizinische Vorrichtung (IMD) (16);
eine oder mehrere Leitungen (18, 20, 22) für eine Erfassungstätigkeit und/oder Abgabe von elektrischer Stimulation von einem Gewebe und/oder einem Organ des Patienten, wobei das eine oder die mehreren Leitungen in dem Patienten implantiert sind und an die implantierbare medizinische Vorrichtung gekoppelt sind; und
einen Prozessor (100) der IMD, der konfiguriert ist um:
eine oder mehr Messungen in Bezug zu einer der implantierten Leitungen durchzuführen;
die Eigenschaften der einen implantierten Leitung basierend auf der einen oder den mehreren Messungen zu bestimmen; und
den Typ der einen implantierten Leitung basierend auf den Leitungseigenschaften zu identifizieren; wobei
die eine oder die mehreren implantierten Leitungen Leitungen mit einem oder mehreren damit integrierten modularen Schaltkreisen umfassen oder Leitungen ohne die modularen Schaltkreise umfassen, wobei jeder der modularen Schaltkreise eine Schnittstelle zwischen Stromleitern und entsprechenden Elektroden der Leitung definiert; und **dadurch gekennzeichnet, dass** die IMD konfiguriert ist, um mit den modularen Schaltkreisen zu funktionieren und dadurch, dass der Prozessor konfiguriert ist, um ein oder mehrere Abfragesignale entlang der Leitungsstromleiter zu übertragen, um entsprechende Reaktionen bzw. In-Kind-Reaktionen zu veranlassen, die von jedem der modularen Stromkreise an den Prozessor übertragen werden und dadurch, dass die modularen Stromkreisen konfiguriert sind, um Reaktionen zu übertragen, die Elektrodenkonfigurationsinformation umfassen, die die modularen Stromkreise betreffen.

10. System nach Anspruch 9, wobei der Prozessor konfiguriert ist, um ein oder mehrere Impedanzwerte der einen implantierten Leitung abzuleiten, wenn die eine oder mehrere Messungen durchgeführt werden.

11. System nach Anspruch 9, wobei der Prozessor konfiguriert ist, um Werte einer elektrischen Variable abzuleiten, wenn die eine oder mehrere Messungen durchgeführt werden, wodurch der Prozessor weiter konfiguriert ist um:
zu bewerten, ob die abgeleiteten Werte der elektrischen Variable innerhalb von gültigen Bereichen der elektrischen Variable fallen, wobei die gültigen Werte der elektrischen Variable innerhalb des Speichers der IMD gespeichert sind; und
die abgeleiteten Werte der elektrischen Variable mit entsprechenden Leitungstypidentifikatoren zu assoziieren, die innerhalb des IMD-Speichers gespeichert sind.

12. System nach Anspruch 11, wobei die elektrische Variable eine Impedanz der implantierten Leitung aufweist, und wobei jeder der Leitungstypidentifikatoren eine Elektrodenkonfiguration der implantierten Leitung aufweist, basierend auf einem entsprechenden Impedanzwert eines Segments der implantierten Leitung.

13. System nach Anspruch 9, weiter aufweisend eine EDV-Vorrichtung (182) in Kommunikation mit der IMD zum Kommunizieren des Typs der implantierten Leitung an einen Nutzer.

## Revendications

1. Procédé pour fournir des informations concernant une sonde implantée pour un dispositif médical implantable, le procédé comportant les étapes consistant à :
réaliser, par un processeur d'un dispositif médical implantable (IMD), une ou plusieurs mesures en liaison avec une sonde implantée couplée à l'IMD ;
déterminer, par le processeur, des caractéristiques de la sonde implantée sur la base de la ou des mesures ; et
identifier, par le processeur, un type de sonde implantée sur la base des caractéristiques de sonde, et dans lequel la sonde implantée comporte un élément parmi une sonde ayant un ou plusieurs circuits modulaires incorporés dans celle-ci et une sonde sans lesdits circuits modulaires, dans lequel chacun des circuits modulaires définit une interface entre des conducteurs et des électrodes correspondantes de la sonde implantée, et **caractérisé en ce que** l'IMD est configuré pour fonctionner avec les circuits modulaires, et **en ce que** le procédé comporte en outre l'étape consistant à :
transmettre un ou plusieurs signaux d'interrogation le long des conducteurs de sonde pour demander des réponses en l'espèce transmises à partir de chacun des circuits modulaires au processeur, dans lequel les réponses comportent des informations de configuration d'électrode se rapportant aux circuits modulaires.

2. Procédé selon la revendication 1, dans lequel la réalisation de la ou des mesures comporte en outre les étapes consistant à :
appliquer des impulsions de stimulation correspondant à des vecteurs de stimulation pour des électrodes de la sonde implantée, dans lequel chacune des impulsions se situe à un seuil non suffisamment significatif pour stimuler un tissu corporel mais à partir duquel un paramètre correspondant peut être détecté ; et
obtenir la ou les mesures en liaison avec les paramètres correspondants.

3. Procédé selon la revendication 2 comportant en outre l'étape consistant à :
sélectionner, par le processeur, les vecteurs de stimulation en liaison avec un coeur.

4. Procédé selon la revendication 1, dans lequel la réalisation de la ou des mesures comporte l'obtention d'une ou plusieurs valeurs d'impédance de la sonde implantée.

5. Procédé selon la revendication 1, dans lequel la réalisation de la ou des mesures comporte l'obtention de valeurs d'une variable électrique de la sonde implantée, et comportant en outre l'étape consistant à :
évaluer, par le processeur, si les valeurs obtenues de la variable électrique se situent dans des plages valides de la variable électrique, les plages valides de la variable électrique étant stockées dans une mémoire de l'IMD.

6. Procédé selon la revendication 5, dans lequel les plages valides de la variable électrique correspondent aux caractéristiques de sonde pour identifier la sonde, dans lequel les caractéristiques de sonde comportent la connectivité des conducteurs de sonde entre l'IMD et des électrodes de la sonde, et le procédé comportant en outre l'étape consistant à :
associer, par le processeur, les valeurs obtenues de la variable électrique à des identifiants de type de sonde correspondants stockés dans la mémoire de l'IMD.

7. Procédé selon la revendication 6, dans lequel la variable électrique comporte une impédance de la sonde implantée, et dans lequel chacun des identifiants de type de sonde comporte une configuration d'électrode de la sonde implantée basée sur une valeur d'impédance correspondante d'un segment de la sonde implantée.

8. Procédé selon la revendication 1, dans lequel la réalisation de la ou des mesures comporte l'obtention d'une variable électrique de la sonde implantée pour différer des agencements actifs des circuits modulaires en liaison avec des électrodes de celle-ci.

9. Système qui facilite l'information concernant une sonde implantée pour un dispositif médical implantable, le système comportant :
un dispositif médical implantable (IMD) (16) ;
une ou plusieurs sondes (18, 20, 22) pour une ou plusieurs actions parmi détecter une activité et délivrer une stimulation électrique d'un ou plusieurs éléments parmi un tissu et un organe du patient, la ou les sondes étant implantées dans le patient et couplées au dispositif médical implantable ; et
un processeur (100) de l'IMD configuré pour :
réaliser une ou plusieurs mesures en liaison avec l'une des sondes implantées ;
déterminer des caractéristiques de la sonde implantée sur la base de la ou des mesures ; et
identifier le type de la sonde implantée sur la base des caractéristiques de sonde,
dans lequel la ou les sondes implantées comportent un élément parmi des sondes ayant un ou plusieurs circuits modulaires incorporés dans celles-ci et des sondes sans lesdits circuits modulaires, dans lequel chacun des circuits modulaires définit une interface entre des conducteurs et des électrodes correspondantes de la sonde ; et **caractérisé en ce que** l'IMD est configuré pour fonctionner avec les circuits modulaires et **en ce que** le processeur est configuré pour transmettre un ou plusieurs signaux d'interrogation le long des conducteurs de sonde pour demander des réponses en l'espèce transmises à partir de chacun des circuits modulaires au processeur, et **en ce que** les circuits modulaires sont configurés pour transmettre des réponses comportant des informations de configuration d'électrode se rapportant aux circuits modulaires.

10. Système selon la revendication 9, dans lequel le processeur est configuré pour obtenir une ou plusieurs valeurs d'impédance de la sonde implantée lors de la réalisation de la ou des mesures.

11. Système selon la revendication 9, dans lequel le processeur est configuré pour obtenir des valeurs d'une variable électrique lors de la réalisation de la ou des mesures, moyennant quoi le processeur est en outre configuré pour :
évaluer si les valeurs obtenues de la variable électrique se situent dans des plages valides de la variable électrique, les plages valides de la variable électrique étant stockées dans une mémoire de l'IMD ; et
associer les valeurs obtenues de la variable électrique à des identifiants de type de sonde correspondants stockés dans la mémoire de l'IMD.

12. Système selon la revendication 11, dans lequel la variable électrique comporte l'impédance de la sonde implantée, et dans lequel chacun des identifiants de type de sonde comporte une configuration d'électrode de la sonde implantée basée sur une valeur d'impédance correspondante d'un segment de la sonde implantée.

13. Système selon la revendication 9, comportant en outre un dispositif de calcul (182) en communication avec l'IMD pour communiquer le type de la sonde implantée à un utilisateur.
